# EUROPEAN PATENT SPECIFICATION

(11) **EP 2 932 901 B1**
(45) Date of publication and mention of the grant of the patent: **26.10.2016**
(21) Application number: 14164837.8
(22) Date of filing: 16.04.2014
(51) Int. Cl.: A61B 6/03, A61B 6/00, H04B 10/00, H04B 5/00

(54) **Rotary joint for multi-channel high speed data transmission**
Drehverbindung für mehrkanalige Hochgeschwindigkeits-Datenübertragung
Joint rotatif pour transmission à haute vitesse de données multi-canaux

(43) Date of publication of application: 21.10.2015
(73) Proprietor: Schleifring und Apparatebau GmbH, 82256 Fürstenfeldbruck (DE)
(72) Inventor: Steffens, Holger, 80993 München (DE); Krumme, Nils, 82340 Feldafing (DE)
(74) Representative: Lohr, Georg

(56) References cited:
- EP-A2- 1 810 617
- US-A- 4 697 183
- US-A- 5 010 254
- US-B2- 7 466 794

## Description

### Field of the invention

The invention relates to capacitive couplers for non-contacting or contactless signal and data transmission, in particular to capacitive rotating transmission devices like rotary joints. Such transmission devices may be used in computer tomography scanners, also called CT scanners.

### Description of the related art

In CT scanners, a rotating x-ray tube and an x-ray detector generate high-speed imaging data. These may be transmitted from the rotating part to the stationary part. Furthermore, control signals for controlling the device and specifically the power supply of the x-ray tube may be transmitted from the stationary to the rotating part. Many applications exist where there is the need to transmit control signals between a rotor and a stator like in windmills, revolving transfer machines, bottling plants, packaging machines or placement heads of insertion machines.

A contactless data link is disclosed in US patent 6,956,450 B1. Here, binary data is fed into a transmission line at the rotating part. The transmission line has a low pass filter characteristic to suppress high frequency noise. It is terminated at its ends with the characteristic impedance of the line. Furthermore, it is split into two segments of equal length, each spanning half the circumference of a rotating structure.

A CT scanner having a sectioned multichannel data link is disclosed in EP 1086 653 B1. The length of the individual transmitting segments causes a delay in the transmitted signal, which is dependent of the rotational angle. To avoid a signal distortion, the phase shift at two adjacent segments must be negligible. This limits the overall bandwidth of the system. This problem is solved in

EP 1810 617 A1 by using split transmission lines, where the individual section of the transmission lines are activated dependent on the rotational angle, such that when a receiver crosses the boundary of neighbored transmission line segments, the same signal is fed into these transmission line segments. The drawback is that for each receiving element, two switched transmitting elements are required. This makes the data link comparatively complex and expensive.

A Multi-channel data link is disclosed in EP 1867 073 B1. This data link uses N transmission lines and N+1 receivers for transmitting N channels.

### Summary of the invention

The problem to be solved by the invention is to provide a multichannel contactless high-speed data link, which may be used in CT scanners, requiring a low number of comparatively simple transmission elements.

Solutions of the problem are described in the independent claims. The dependent claims relate to further improvements of the invention.

The invention is based on the finding that complex transmission line structures in contactless data links increase the costs and reduce reliability. As the transmission line must handle very high frequencies in the range of several GHz, this is a critical RF component. Cabling and the electrical connections must be long time stable and provide the RF performance over a long time.

A basic contactless data link for transferring data from a rotating part to a stationary part has at least one transmission line into which the signals are fed, the transmission line being arranged at the rotating part. Preferably, a transmitter amplifier, which receives the signals to be transmitted, is provided for feeding the signals into a transmission line, preferably into a first end of a transmission line. It is preferred, if the opposite end of the transmission line is terminated by a termination. For receiving the signals, at least a receiving coupler is provided which preferably is arranged movable in close proximity to the transmission line. For amplifying and optionally reconstructing or retiming the received signals, a receiving amplifier may be provided. The output signal of the receiving amplifier may be forwarded to a data processing unit. For clarification, the feeding points are the points to which transmitter amplifiers are connected to a transmission line. The feeding points may be at the center of a transmission line. Most preferably, each transmission line is connected at one end to a transmitter amplifier and at its other end to a termination, which absorbs the signal propagating through the transmission line and prevents a reflection of these signals. The transmitter amplifiers may be integrated into or be part of any other transmitter component. Similarly, the receiving amplifiers may be integrated into or be part of any other receiver component.

In a preferred embodiment, an N channel data link can be built with N+1 transmission line segments, wherein N is a positive integer greater than 1, preferably greater than 2. Most preferably, N is an odd number, which allows to have an even number of transmitting lines, providing minimum phase differences between adjacent transmitting lines. It is further preferred to have at least two feeding points at adjacent transmission lines at their adjacent ends. It is further preferred to have at least two terminations of adjacent transmission lines at their adjacent ends. It is most preferred to have an even number of transmission lines and the feeding points of adjacent transmission lines arranged at the adjacent ends and the terminations of adjacent transmission lines terminated at the adjacent ends. This results in a sequence of adjacent feeding points and adjacent terminations.

When the rotating part rotates against the stationary part, the position of the receiving couplers against the transmission lines changes continuously. Accordingly, the input signals of each channel must be routed to the specific transmission line, which is opposed by the receiving coupler of the corresponding output channel, coupling signals into the receiving coupler. This routing must be done in dependence of the relative position between the rotating and the stationary part. As the number of transmission lines (N+1) is larger than the number of receiving couplers (N), there preferably at each time is one transmission line, which is not opposed by any receiving coupler. Preferably, changing of the channel assignment of a transmission line is done in this case, when no receiving coupler is coupled to this transmission line. This allows a switching of channels to adapt for the rotation without incurring any loss of data. The position of the receivers can deviate of their ideal position of equal distance or angle to a certain amount so that always enough time remains for the switch to switch the channel of the unused transmission link and allow for an adjustment of the delay units and clock synchronizers. This allows to adapt to mechanical restrictions in the system. For triggering the switching process, a position encoder indicating the relative position between rotating and stationary part is required. It is preferred, if this is a simple encoder for encoding N+1 positions associated to each of the transmission lines. Preferably, the encoder provides a trigger signal just after a receiving coupler has left a certain transmission line or just before a receiving coupler is going to move on a transmission line. In an alternate embodiment a high-resolution position indicator, which is available in each CT scanner may be used.

Due to the position-dependent routing of the input signals, basically the same signals are delivered to adjacent transmission lines at the time, when a receiving coupler passes from one transmission line to another transmission line. Approximately, at the center between a first and a second neighbored transmission line, the receiving coupler receives the signals from both transmission lines with the same amplitude. If the signals have a phase shift relative to each other, the sum of both signals as received by the receiving coupler may appear as a distorted signal. Such a signal distortion results in a loss of data and may require a retransmission of a data frame. Therefore, it is essential that the phase shift between the signals at adjacent transmission lines are minimized, at least at the time when a receiving coupler is passing from one transmission line to another transmission line. Such a minimizing of phase or timing differences can be done by a good selection of the components, taking care that the propagation times of the circuits like the transmitter amplifiers are equal, the connection cables have the same lengths and the transmission lines also have the same length and the same propagation speed. All this is a complicated process and requires significant amount of work causing significant costs. There may be further deviations caused by aging or variations in temperature. To minimize the phase shift between adjacent transmission lines, it is therefore preferred to have at least one delay unit for adjusting the delay of signals fed into adjacent transmission lines. It may be sufficient to connect such a delay unit only to one of two adjacent transmission lines, adapting the delay of the signals fed into one transmission line to the delay of signals fed into the other transmission line. In a preferred embodiment, a delay unit is provided with each transmission line, which allows the most flexible adjustment of delays. This means that generally, N delay units would be sufficient, although it is preferred to have N+1 delay units.

In a further preferred embodiment, there is a calibration procedure for adjusting of the delays of the delay units. Such a calibration procedure may be triggered each time after turn-on of the device, or in certain time intervals, preferably when the unit is not in use, e.g. if a CT scanner is not performing a scan and runs in idle mode. A calibration may also be triggered if a bit error rate or any other signal or transmission quality parameter detected exceeds a certain threshold.

It is further preferred to use a specific bit pattern transmitted over the data link for calibration. There may be a different bit pattern for each of the data channels to verify the assignments of the channels and/or for checking of crosstalk between the channels.

It is further preferred, if the calibration comprises at least two phases having different synchronization ranges. A first phase may have a broad range of synchronization, and therefore it is preferred to have a bit pattern transmitted over the data link having a low number of 0-1 transitions, which may for example be a pattern like 000111. Following this first calibration phase, preferably at least a second calibration phase for a more precise calibration, but providing a smaller synchronization range may be provided. For this purpose, another bit pattern having a higher number of 0-1 transitions may be provided. It is preferred, if a pseudo noise pattern like a PRBS31 pattern may be used.

Preferably, each calibration phase is performed by setting a certain delay time to a delay unit and rotating the rotating part against the stationary part. The number of bit errors detected, preferably, when a receiving antenna passes from a first to a second transmission line is recorded. Alternatively, any other signal quality parameter like a bit error rate may be recorded. This procedure is repeated with different settings of the delay units. These settings may be stored in a table or calculated before or during the calibration. According to the test results, a setting of a delay unit providing a good or even the best transmission quality or bit error rate is selected. The results of the calibration provide delay settings for later operation of the unit.

In a perfectly adjusted unit, the sum of all delays over a rotation (360 degrees) is zero. In actual systems, there may be cases where the sum of all delays over a rotation has a positive or negative value, which would cause summing up to high values during several rotations. These high values may exceed the maximum range of the delay units. To avoid this problem, in low speed systems with a low data rate, the delay values may be calculated such that the sum of all delay values over a rotation is zero. This may cause a deviation from the optimum delay value measured during calibration, which may be acceptable for lower data rates, which may be in a range of approximately below 600 Mb/sec. For higher data rates, it is preferred to adjust the delay continuously or in small steps after a receiving coupler has passed from a first transmission line to a second transmission line. During the time, the receiving coupler is moving over the second transmission line, receiving signals from the second transmission line, the delay of the signals fed into the second transmission line may be readjusted to a certain nominal value to avoid summing up of delays.

The description herein relates to the transmission of signals and/or data from a rotating part to a stationary part, which corresponds to a preferred application in CT scanners. It is obvious, that the concepts provided herein may also be applied to transfer data from a stationary to a rotating part, or even between parts, which may be movable by a linear motion instead of a rotation.

Forward Error Correction (FEC) technologies as well as backward error correction (BEC) can be implemented in the protocol and even combined FEC and BEC. In the case of a BEC a back channel for the request of distorted messages has to be added.

In some applications like CT scanners, a bidirectional data link may be required. Here, the N channel data link disclosed herein comprising N+1 transmission line segments and N receiving couplers may be used for transferring data in a first direction, like from a rotating part to a stationary part. Preferably, a second N channel data link is used for transferring data in an opposite direction. Most preferably, the second data link comprises N transmission line segments and N+1 receiving couplers. This allows a comparatively simple assembly, for example having N+1 components (N+1 transmission line segments in first direction and N+1 receiving couplers in second direction) at the rotating part and having N components (N transmission line segments in second direction and N receiving couplers in first direction) at the stationary part.

Although the preferred embodiment of the invention relate to a data link for CT scanners and a CT scanner having such a data link, the embodiments may be used in any other application, where a high-speed multichannel data link is required. Such applications may be any other scanner, which for example may use a microwave scanning system, or any scanner for example for scanning of luggage or other materials, like wood, plastic or composite structures.

A further aspect of the invention relates to a method for transferring data between a rotating and a stationary part, as described herein.

Further aspects of the invention relate to rotary joints for CT scanners and CT scanners comprising rotary joints as described herein.

### Description of Drawings

In the following, the invention will be described by way of example, without limitation of the general inventive concept, on examples of embodiment with reference to the drawings.
- Figure 1: shows a schematic diagram of the invention.
- Figure 2: shows the timing relationship between transmission lines and receiving couplers.
- Figure 3: shows further timing details.
- Figure 4: shows a calibration result table.

- Figure 5: shows an exemplary CT scanner.

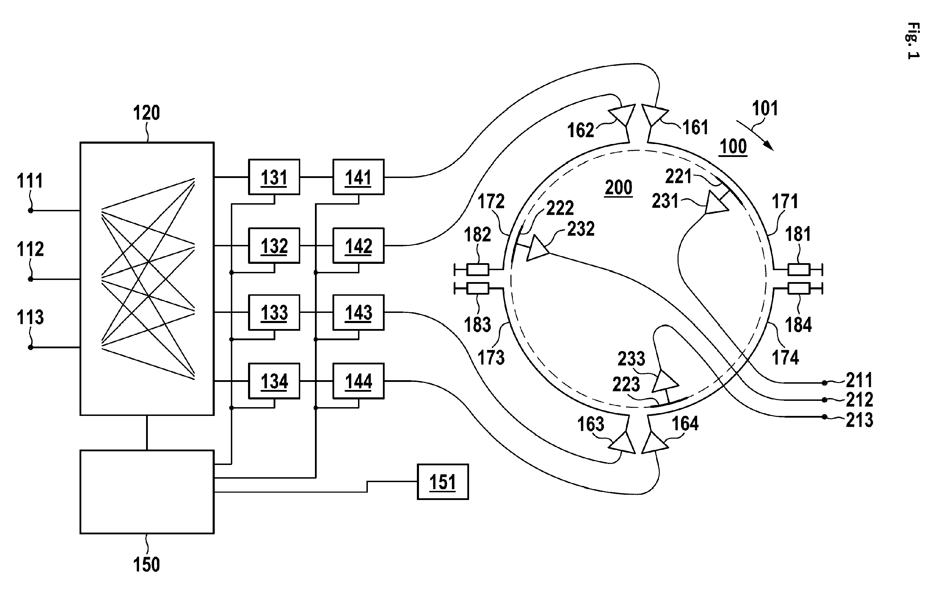

In Figure 1, a schematic diagram of a preferred embodiment of the invention is shown.

A rotary joint is provided for coupling data from a rotating part 100 to a stationary part 200. In this specific embodiment, the direction 101 of the rotating part 100 is clockwise, although a counter-clockwise rotation is also possible.

Input data is received for example from a DAS (data acquisition system) via a first input channel 111, a second input channel 112 and a third input channel 113 on the rotating part 100. This data has to be coupled to a first output channel 211, a second output channel 212, and a third output channel 213 at the stationary part 200. In this embodiment, the number of channels N is equal to 3, although any other number of channels may be selected. The input channels are coupled via a routing means, which preferably is a crosspoint switch 120 to N+1 transmission paths, which equals to 4 transmission paths in this embodiment. The crosspoint switch 120 is preferably controlled by a controller 150. Each transmission path preferably comprises a delay unit 141, 142, 143, 144, preferably receiving the signals from clock synchronizers 131, 132, 133, 134, being fed by the routing means. The clock synchronizers synchronize the clock of the data streams received the input channels. They also may compensate for different delay times caused by the routing means. Therefore, all signals have the same timing. The delay units provide variable delays to the signals. The clock synchronizers may also be omitted, if the timing relationship of the input signals is known and comparatively constant. In this case, the delays of the delay unit may be adjusted accordingly. Preferably, the clock synchronizers receive a common clock which may be delivered by a controller 150, and which is used to synchronize the signals of the data channels. Furthermore, the delay units may also be controlled by control unit 150, which sets the delay units to position-dependent delay values. For this purpose, a position sensor 151 may be provided, detecting the relative position between stationary and rotating parts. The signals from the delay units are delivered to transmitter amplifiers 161, 162, 163, 164, feeding transmission lines 171, 172 173, 174, preferably at one end. Furthermore, the transmitter amplifiers may do signal forming, like a pre-emphasis, and/or adapting the signals to the transmission line characteristics. The transmission lines preferably are arranged at the rotating part, such that they cover almost the whole circumference. At the ends opposing to the transmitter amplifiers, preferably terminations 181, 182, 183, 184 are provided. It is most preferred, if first adjacent ends of adjacent transmission lines are each coupled to a transmitter amplifier, while the opposite ends are coupled to terminations. At the stationary part, there are N receiving couplers 221, 222, 223, the receiving couplers moving in close proximity to the transmission lines for picking up signals of the transmission lines. The signals from the receiving couplers are forwarded by receiving amplifiers 231, 232, 233 for amplifying and/or shaping and/or retiming the signals to output channels 211, 212, 213.

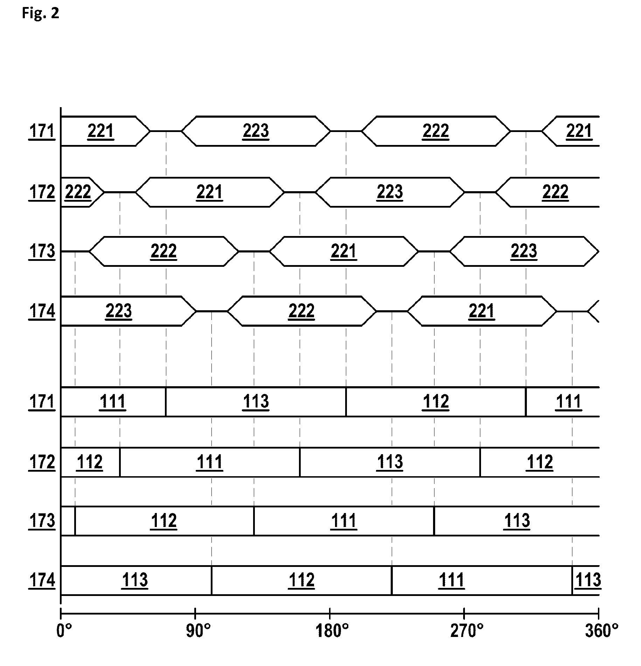

In Figure 2, the relationship between transmission lines, receiving couplers, and the signals coupled therein are shown. All diagrams show from left to right the status starting at an angle of 0° corresponding to the angular position as shown in Figure 1, while the rotating part moves clockwise against the stationary part. The diagram ends at the rightmost position at a rotation angle of 360°, corresponding to a full rotation. The first four rows show the interaction of receiving couplers with the transmission lines. The first row relates to the first transmission line 171, the second to the second transmission line 172, the third to the third transmission line 173, and the fourth to the fourth transmission line 174. The next four rows indicate the signals, which are coupled into each of the transmission lines 171-174.

Coming back to the first four rows, starting with the 0° position as shown in Figure 1, first receiving coupler 221 receives signal from transmission line 171, while the second receiving coupler 222 receives signals from second transmission line 172. The third transmission line 173 is not coupled to any receiving coupler, and the fourth transmission line 174 couples the signals to receiving coupler 223. How the receiving couplers interact with transmission lines during rotation is further explained in detail with respect to transmission line 171, corresponding to the topmost row. When starting with an angle of 0°, the first transmission line 171 interacts with first receiving coupler 221. After some rotation, the receiving coupler 221 leaves transmission line 171, causing a short period of time where no receiving coupler receives a signal from said transmission line. At a further degree of rotation, the third receiving coupler 223 enters into the range of a first transmission line 171. It later leaves the range of said transmission line and is being followed by the second receiving coupler 222. Finally, signals are coupled over to the first receiving coupler 221 until a full rotation has been performed. After 360° of rotation, the process starts again with 0° of rotation, as explained before. Similar interactions between transmission lines and receiving couplers occur for the other transmission lines. Due to the higher number (N+1) of transmission lines compared to the number N of the receiving couplers, there is always a gap, where no receiving coupler interacts with a transmission line. In this gap, the routing of signals is adjusted, as is shown in the lower four rows of the diagram, which is exemplarily explained by the relationship of transmission line 171.

Moving to the lower four rows of the diagram, during the time, where the first receiving coupler 221 interacts with transmission line 171, the signal of input channel 111 is routed by the crosspoint switch 120 to said transmission line. When receiving coupler 223 interacts with said transmission line, data of input channel 113 is routed to said transmission line. Furthermore, when receiving coupler 222 interacts with said transmission line, data of input channel 112 is routed to said transmission line. Switching of the data is always performed in the time gap, where no data of the transmission line is received by a receiving coupler, therefore causing no loss of data. It is preferred, if switching is done at the center of the time gap. To reduce EMI, during the time gap, transmission may be suspended. Similar function applies to the other transmission lines as shown by the following rows in the diagram.

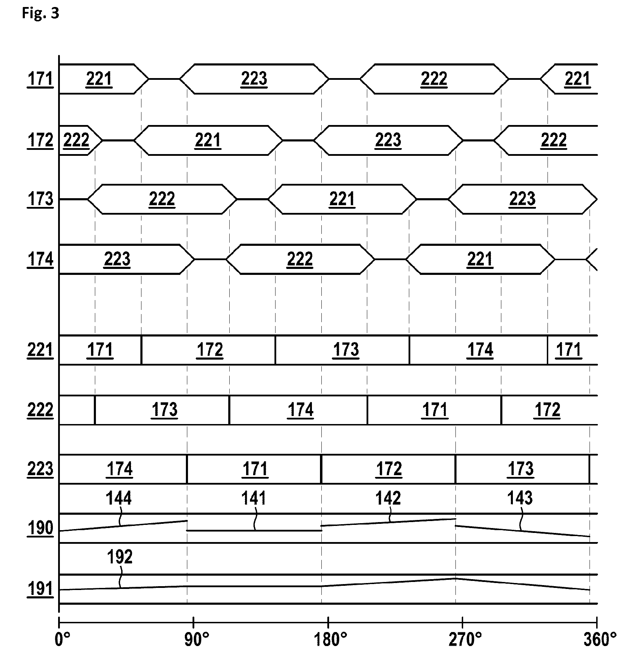

Figure 3 shows further timing details. The topmost four rows are the same as in the previous figure. The next three rows below show the assignment of transmission lines to receiving couplers. First, receiving coupler 221 receives signals from transmission line 171. It is then handed over to transmission lines 172, 173, 174 and back to 171. Receiving coupler 222 starts with receiving signals from transmission line 172 and is further forwarded to transmission lines 173, 174 and 171 back to 172. Receiving coupler 223 starting with transmission line 174 is handed over to transmission lines 171, 172 and 173, going back to 174.

The last two rows show delay times with respect to receiving coupler 223. In row 190, the delay times of the delay units 144, 141, 142 and 143 are shown during the time intervals, when receiving coupler 223 is receiving signals from transmission lines 174, 171, 172 and 173. In row 191, the curve 192 shows the resulting absolute phase or delay of the signal received by receiving coupler 223. This is only an exemplary embodiment. It is obvious, that depending on the physical structures and characteristics of the parts different delays may result. Similar curves apply also for the other receiving couplers.

Generally, when moving over a transmission line, a receiving coupler will receive signals with increasing or decreasing delay. This delay is caused by a change of the distance of the receiving coupler relative to a transmission line feeding point coupled to a transmitter amplifier. Due to the transmission line arrangement with alternating pairs of feeding points and terminations, this kind of delay causes no signal loss in a perfectly adjusted system, as the delay times at the pairs of feeding points and terminations are the same. In addition to this delay, there may be a further delay caused unequal runtimes in the components as described above. In most cases this further delay is much less than the delay caused by a change of the distance. Therefore, the following diagrams show only the further delay. As described above, the delay times of the delay units are adjusted such, that a receiving coupler notices no or a negligible phase difference or delay in signals between neighboring transmission lines, when passing from one transmission line to another. This is shown by curve 192, where the resulting phase or delay of the signal as received by receiving coupler 223 does not change at transitions between transmission lines. For obtaining this, the delays of the delay units must be set accordingly. As the resulting delays of curve 192 are the sum of the delay of the delay units and the delay caused by the remaining signal path to the transmission lines including cabling and transmitter amplifiers, the individual delay curves 144, 141, 142 and 143 look different from curve 192. There may even be different delays in neighboring transmission lines.

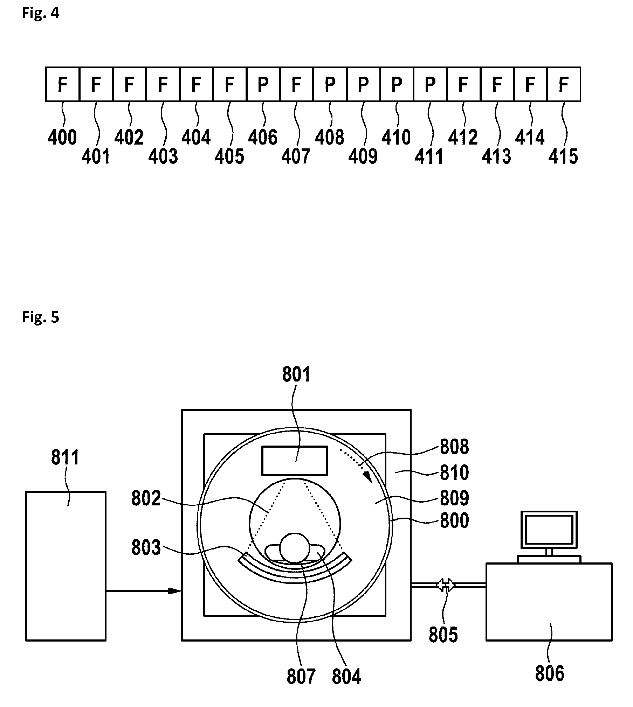

Figure 4 shows an embodiment of a calibration test result table. As described above in detail, a calibration may be performed for determining the optimum delay values for the delay units dependent of the angular position, and most preferably for position corresponding to the gaps between adjacent transmission lines. Here, measurements may be performed by setting a delay element to different delay values and measuring the resulting bit error rate or any other characteristic value during rotation, preferably during rotation over a gap between two adjacent transmission lines. The table shows the results 400 -415 of a series of tests with varying delay settings. The result 400 may be the result of a lowest delay setting, while the result 415 may come from the highest delay setting. Preferably, the delay setting between the results 400 and 415 is linearly increased. In this example, the results 400 -405 indicate "failed" by a letter F, for example by exceeding a certain bit error rate threshold. The result of 406 is "passed" indicated by letter P, as in this example the bit error rate was below the threshold. The result 407 was again failed, while the results 408 -411 are passed. Again, the results for delays 412-415 are failed. Here, preferably a centered result in the field of the passed fields is selected. In this embodiment, the preferred delay setting would be the delay setting used for obtaining result 409.

Figure 5 shows schematically a CT scanner gantry. The stationary part is suspended within a massive frame 810. The rotating part 809 of the gantry is rotatably mounted with respect to the stationary part and rotates along the rotation direction 808. It supports an X - ray tube 801 for generating an X-ray beam 802 that radiates through a patient 804 lying on a table 807 and is intercepted by a detector 803 and converted to electrical signals and imaging data thereof. Electrical power from power supply unit 811 may be transmitted by a slipring (not shown) to the rotating part. The data obtained by the detector 803 are transmitted via high-speed network contactless rotary joint 800 to an evaluation unit 806 by means of a data bus or network 805. Is obvious, that the application of the invention is not limited to CT scanners.

### List of reference numerals

- 100: rotating part
- 101: direction of rotation
- 111-113: input channels 1 - 3
- 200: stationary part
- 211-213: output channels 1- 3
- 221-223: receiving couplers 1 - 3
- 231-233: receiving amplifiers 1- 3
- 120: crosspoint switch
- 131-134: clock synchronizers 1 - 4
- 141-144: delay units 1-4
- 150: controller
- 151: position sensor
- 161-164: transmitter amplifiers 1 - 4
- 171-174: transmission lines 1 - 4
- 181-184: terminations 1 - 4
- 190: delays of delay units
- 191: delays at receiving coupler
- 192: delay curve
- 400-415: test results for varying delay times
- 800: contactless rotary joint
- 801: x-ray tube
- 802: x-ray beam
- 803: x-ray detector
- 804: patient
- 805: network
- 806: evaluation unit
- 807: patient table
- 808: rotation direction
- 809: rotating part
- 810: frame
- 811: power supply unit

## Claims

1. Device for transferring data of N data channels from a rotating part (100) to a stationary part (200), the rotating part comprising:
- N input channels (111, 112, 113) for receiving data of the N data channels,
- a routing means (120) for routing dependent of the relative position of the rotating part (100) to the stationary part (200) the signals of the input channels (111, 112, 113) to N+1 transmission lines via
- delay units (141, 142, 143, 144) and transmitter amplifiers (161, 162, 163, 164),
the stationary part comprising N receiving couplers (221, 222, 223) connected to N receiving amplifiers (231, 232, 233) for delivering the signals to N output channels (211, 212, 213).

2. Device according to claim 1,
**characterized in, that**
between the routing means (120) and the delay unit (141, 142, 143, 144), clock synchronizers (131, 132, 133, 134) are provided.

3. Device according to claim 1 or 2,
**characterized in, that**
a controller (150) is provided for controlling the delay units (141, 142, 143, 144).

4. Device according to claim 3,
**characterized in, that**
the controller provides delay values to the delay units (141, 142, 143, 144) dependent on the relative position between rotating and stationary part.

5. Device according to any one of the preceding claims,
**characterized in, that**
a position sensor is provided delivering information on the relative position between rotating and stationary part.

6. Device according to any one of the preceding claims,
**characterized in, that**
the transmission lines are connected with a first end to the transmitter amplifiers (161, 162, 163, 164) and with a second end to terminations (181, 182, 183, 184).

7. Device according to any one of the preceding claims,
**characterized in, that**
the transmitter amplifiers (161, 162, 163, 164) of adjacent transmission lines (171, 172, 173, 174) are connected to the adjacent ends of said transmission lines.

8. Device according to any one of the preceding claims,
**characterized in, that**
the controller (150) performs a learning procedure for learning optimum delay times for the delay units (141, 142, 143, 144).

9. Device according to claim 8,
**characterized in, that**
the learning procedure is triggered after power-on of the device, and/or by predetermined time intervals and/or by exceeding a transmission error threshold.

10. Method for transmitting data between a rotating and a stationary part, by receiving data via N input channels (111, 112, 113), forwarding the data via a routing means (120) and via N+1 delay units (141, 142, 143, 144) to N+1 transmission lines (171,172,173,174), further coupling the signals to N receiving couplers (221, 222, 223) and forwarding the received signals to N output channels (211, 212, 213).

11. Method according to claim 10,
**characterized in, that**
the delay times of the delay units (141, 142, 143, 144) are selected dependent of the relative position between rotating and stationary parts.

12. Rotary joint for a CT scanner, comprising a device according to any one of claims 1-9.

13. CT scanner comprising a rotary joint according to claim 12.

## Patentansprüche

1. Vorrichtung zum Übertragen von Daten von N Datenkanälen von einem rotierenden Teil (100) an einen stationären Teil (200), wobei der rotierende Teil umfasst:
- N Eingangskanäle (111, 112, 113) zum Empfangen von Daten der N Datenkanäle,
- ein Routing-Mittel (120) zum Routing der Signale der Eingangskanäle (111, 112, 113) an N+1 Übertragungsleitungen, abhängig von der relativen Position des rotierenden Teils (100) zu dem stationären Teil (200), über
- Verzögerungseinheiten (141, 142, 143, 144) und Sendeverstärker (161, 162, 163, 164),
wobei der stationäre Teil N Empfangskoppler (221, 222, 223) umfasst, welche mit N Empfangsverstärkern (231, 232, 233) verbunden sind, um die Signale an N Ausgangskanäle (211, 212, 213) zu liefern.

2. Vorrichtung nach Anspruch 1,
**dadurch gekennzeichnet, dass**
zwischen dem Routing-Mittel (120) und der Verzögerungseinheit (141, 142, 143, 144), Taktsynchronisatoren (131, 132, 133, 134) vorgesehen sind.

3. Vorrichtung nach Anspruch 1 oder 2,
**dadurch gekennzeichnet, dass**
eine Steuerung (150) zum Steuern der Verzögerungseinheiten (141, 142, 143, 144) vorgesehen ist.

4. Vorrichtung nach Anspruch 3,
**dadurch gekennzeichnet, dass**
die Steuerung den Verzögerungseinheiten (141, 142, 143, 144) Verzögerungswerte in Abhängigkeit von der relativen Position zwischen rotierendem und stationärem Teil bereitstellt.

5. Vorrichtung nach einem der vorhergehenden Ansprüche,
**dadurch gekennzeichnet, dass**
ein Positionssensor vorgesehen ist, welcher Informationen über die relative Position zwischen rotierendem und stationärem Teil liefert.

6. Vorrichtung nach einem der vorhergehenden Ansprüche,
**dadurch gekennzeichnet, dass**
die Übertragungsleitungen mit einem ersten Ende mit den Sendeverstärkern (161, 162, 163, 164) und mit einem zweiten Ende mit Abschlüssen (181, 182, 183, 184) verbunden sind.

7. Vorrichtung nach einem der vorhergehenden Ansprüche,
**dadurch gekennzeichnet, dass**
die Sendeverstärker (161, 162, 163, 164) von benachbarten Übertragungsleitungen (171, 172, 173, 174) an die benachbarten Enden der Übertragungsleitungen verbunden sind.

8. Vorrichtung nach einem der vorhergehenden Ansprüche,
**dadurch gekennzeichnet, dass**
die Steuerung (150) einen Lernverfahren zum Lernen optimaler Verzögerungszeiten für die Verzögerungseinheiten (141, 142, 143, 144) ausführt.

9. Vorrichtung nach Anspruch 8,
**dadurch gekennzeichnet, dass**
der Lernverfahren nach dem Einschalten der Vorrichtung, und/oder durch vorbestimmte Zeitintervalle und/oder durch Überschreiten eines Sendefehler-Schwellenwertes ausgelöst wird.

10. Verfahren zu Senden von Daten zwischen einem rotierenden und einem stationären Teil, durch Empfangen von Daten über N Eingangskanäle (111, 112, 113), Weiterleiten der Daten über ein Routing-Mittel (120) und über N+1 Verzögerungseinheiten (141, 142, 143, 144) an N+1 Übertragungsleitungen (171, 172, 173, 174), weiterhin Koppeln der Signale an N Empfangskoppler (221, 222, 223) und Weiterleiten der empfangenen Signale an N Ausgangskanäle (211, 212, 213).

11. Verfahren nach Anspruch 10, **dadurch gekennzeichnet, dass** die Verzögerungszeiten der Verzögerungseinheiten (141, 142, 143, 144) abhängig von der relativen Position zwischen rotierendem und stationärem Teilen ausgewählt werden.

12. Drehübertrager für einen CT-Scanner, umfassend eine Vorrichtung nach einem der Ansprüche 1 - 9.

13. CT-Scanner umfassend einen Drehübertrager nach Anspruch 12.

## Revendications

1. Un dispositif pour transférer des données de canaux de données N à partir d'une partie rotative (100) vers une partie stationnaire (200), la partie rotative comprenant :
- des canaux d'entrée N (111, 112, 113) pour recevoir des données des canaux de données N,
- un moyen d'acheminement (120) pour acheminer en fonction de la position relative de la partie rotative (100) vers la partie stationnaire (200) les signaux des canaux d'entrée (111, 112, 113) vers des lignes de transmission N+1 via
- des unités de retardement (141, 142, 143, 144) et des amplificateurs de transmetteur (161, 162, 163, 164),
La partie stationnaire comprenant des coupleurs de réception N (221, 222, 223) connectés à des amplificateurs de réception N (231, 232, 233) pour émettre les signaux sur des canaux de sortie N (211, 212, 213).

2. Un dispositif selon la revendication 1,
**Caractérisé en ce que**
Entre le moyen d'acheminement (120) et l'unité de retardement (141, 142, 143, 144), des synchroniseurs d'horloge (131, 132, 133, 134) sont fournis.

3. Un dispositif selon la revendication 1 ou 2,
**Caractérisé en ce que**
Un contrôleur (150) est fourni pour contrôler les unités de retardement (141, 142, 143, 144).

4. Un dispositif selon la revendication 3,
**Caractérisé en ce que**
Le contrôleur fournit des valeurs de retardement aux unités de retardement (141, 142, 143, 144) en fonction de la position relative entre la partie rotative et la partie stationnaire.

5. Un dispositif selon l'une des revendications précédentes,
**Caractérisé en ce que**
Un capteur de position est fourni pour donner des informations sur la position relative entre la partie rotative et la partie stationnaire.

6. Un dispositif selon l'une des revendications précédentes,
**Caractérisé en ce que**
Les lignes de transmission sont connectées avec une première extrémité aux amplificateurs du transmetteur (161, 162, 163, 164) et avec une deuxième extrémité aux terminaisons (181, 182, 183, 184).

7. Un dispositif selon l'une des revendications précédentes,
**Caractérisé en ce que**
Les amplificateurs du transmetteur (161, 162, 163, 164) des lignes de transmission adjacentes (171, 172, 173, 174) sont connectés aux extrémités adjacentes desdites lignes de transmission.

8. Un dispositif selon l'une des revendications précédentes,
**Caractérisé en ce que**
Le contrôleur (150) effectue une procédure d'apprentissage pour apprendre des durées de retard optimums pour les unités de retardement (141, 142, 143, 144).

9. Un dispositif selon la revendication 8,
**Caractérisé en ce que**
La procédure d'apprentissage est déclenchée après la mise sous tension du dispositif, et/ou par des intervalles de temps prédéterminés et/ou en dépassant un seuil d'erreur de transmission.

10. Une méthode pour transmettre des données entre une partie rotative et une partie stationnaire, en recevant des données via des canaux d'entrée N (111, 112, 113), envoyant les données via un moyen d'acheminement (120) et via des unités de délai N+1 (141, 142, 143, 144) à des lignes de transmission N+1 (171, 172, 173, 174), couplant de plus les signaux aux coupleurs de réception N (221, 222, 223) et envoyant les signaux reçus aux canaux de sortie N (211, 212, 213).

11. Une méthode selon la revendication 10,
**Caractérisée en ce que**
Les durées de retard et les unités de retardement (141, 142, 143, 144) sont sélectionnées en fonction de la position relative entre les parties rotatives et les parties stationnaires.

12. Un joint rotatif pour un scanner CT, comprenant un dispositif selon l'une des revendications 1 à 9.

13. Un scanner CT comprenant un joint rotatif selon la revendication 12.
